# EUROPEAN PATENT SPECIFICATION

(11) **EP 2 977 015 B1**
(45) Date of publication and mention of the grant of the patent: **25.08.2021**
(21) Application number: 15177465.0
(22) Date of filing: 20.07.2015
(51) Int. Cl.: A61B 17/32, A61B 17/16

(54) **MEDICAL INSTRUMENT FOR REMOVAL OF ARTICULAR CARTILAGE**
MEDIZINISCHES INSTRUMENT ZUM ENTFERNEN VON GELENKKNORPEL
INSTRUMENT MÉDICAL D'ÉLIMINATION DU CARTILAGE ARTICULAIRE

(30) Priority: 21.07.2014 PL 40893814
(43) Date of publication of application: 27.01.2016
(73) Proprietor: Biovico Sp. z o.o., 81-061 Gdynia (PL)
(72) Inventor: SADLIK, Boguslaw, 50-505 Wroclaw (PL)
(74) Representative: Matyka, Malgorzata

(56) References cited:
- CN-Y- 2 330 318
- US-A- 4 242 758
- US-A1- 2008 243 126
- US-A1- 2010 100 097
- None

## Description

The subject of invention is the medical instrument for removal of articular cartilage for using in arthroscopic knee surgery, especially for removal of damaged articular cartilage in area of closer edge of defect, e.g. from the side of insertion of the instrument to the joint.

There are two primary indications for removal of articular cartilage. The first one is the removal of unstable parts of cartilage revealed during arthroscopy. It prevents the mechanical irritation of the joint, which could lead to inflammation and cause the pain. This isolated procedure do not ensure the fulfillment of damage by cartilaginous regenerate. In the second case the procedure of removal of articular cartilage, called chondrectomy, is the preparation of the cartilage damage for the biological reconstruction, which intends to fulfil it by cartilaginous or cartilage-like tissue.

The first, crucial stage of repairing of damaged cartilage surface is surgical debridement of cartilaginous edges and site of damage, e.g. defect of cartilage. The correct performance of this procedure needs the formation of even, stable edges of cartilage defect within the healthy cartilaginous tissue, which are perpendicular to the joint surface and reach the bone bottom of the defect. The exact removal of necrotic tissue is also essential, as novel biological tissue fulfilment should establish the permanent junction with bone surface of defect's bottom.

The most frequent instrument used for cartilage defect debridement during open as well as arthroscopic procedures is a bone curette, which comprises a metal bar element equipped with the handle on the one side and workpiece with cutting edge on the opposite one. The instruments described above are widely used but they do not allow to achieve satisfactory quality of cartilage defect debridement during arthroscopic, e.g. minimally invasive surgical procedure. Document US2010/100097 A1 discloses an instrument that forms the basis of the preamble of claim 1.

The scope of the invention is defined by claim 1. The goal of the solution according to the invention is development of the construction of that instrument, which could make possible during arthroscopy fast and effective creating of stable edges around of whole cartilage defect, perpendicular to the bone surface.

The subject of the invention is a medical instrument for removal of articular cartilage in the form of metal bar element equipped with the handle on the one end and a workpiece with a frontal cutting edge on the opposite one, the frontal cutting edge being located on the leading edge and across the width of the frontal wall, and the workpiece comprising a hook-like protuberance with a profile similar to isosceles triangle of 3-7 mm width and 3-6 mm height, which frontal wall is arched and concaved by the radius of curvature of R=3-10 mm, where the hooked end comprising the workpiece frontal wall is transverse and comprises the frontal cutting edge and longitudinal cutting edges.

This is very advantageous when the workpiece-end of the instrument is arched, with the convexity directed to the frontal edge of workpiece, which facilitates the fitting of workpiece to the oval of defect's wall.

Medical instrument according to the invention allows in the area of closer edge of defect a formation of even, stable cartilage edge with walls perpendicular to the joint surface, thereby to properly prepare the bottom of the defect. This is possible without the need of performance of additional accesses to the joint, so called arthroscopic portals, which is necessary when using bone curette. Therefore there is no additional incisions of the skin, and the procedure could be performed regardless anatomical limitations in all location inside the joint. Important benefit is the inflexibility of the instrument, resulting from hook-like protuberance form of workpiece with the profile similar to isosceles triangle, which finally facilitates the scraping off the solid, calcified cartilage from bone bottom of the defect.

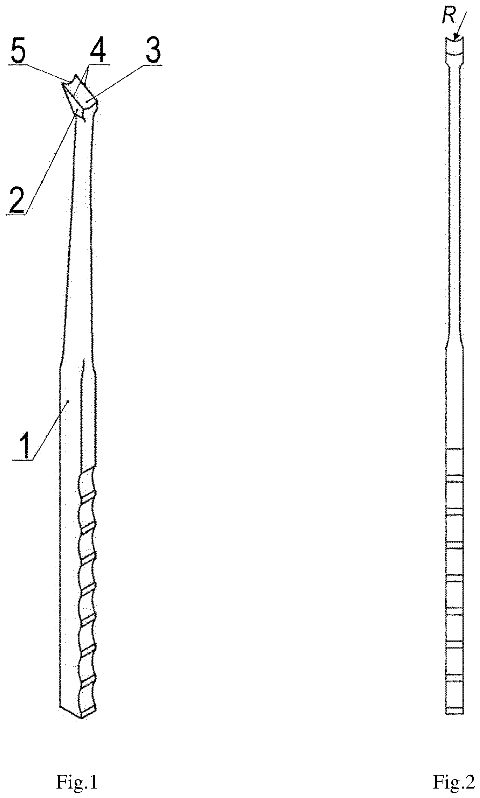

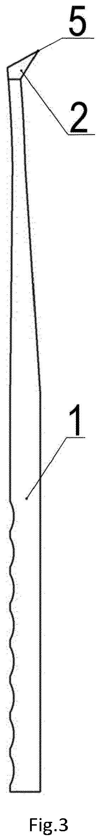

The subject of the invention is showed on the Figures - Fig. 1 presents the medical instrument in axonometric projection at the front position, Fig. 2 presents the medical instrument at the front position and Fig. 3 - at the lateral position.

The medical instrument comprises a metal bar element equipped with the handle (1) on the one side and workpiece (2) with cutting edge on the opposite one. The workpiece (2) comprises the hook-like protuberance with the profile similar to isosceles triangle, which width is 4,5 mm and height is 5 mm. The frontal wall of protuberance is arched and concaved by the radius of curvature of R=5 mm. The longitudinal edges (4) of frontal wall (3) and its frontal edge (5) are the cutting edges.

The height of workpiece - protuberance is determined by the width of the space within the joint and the thickness of the cartilage, while its width is adjusted to the most frequently circumference of proximal edge of cartilage defect. Workpiece-end of the instrument is arched and concaved, with the convexity directed to the frontal edge (5) of workpiece (2).

The instrument is characterized by angle of attack of 120°, which is determined by direction of instrument insertion by incision of the skin.

## Claims

1. A medical instrument for removal of articular cartilage in the form of metal bar element equipped with the handle (1) on the one end and a workpiece (2) with a frontal cutting edge (5) on the opposite one, the frontal cutting edge (5) being located on the leading edge and across the width of the frontal wall (3), and the workpiece (2) comprising a hook-like protuberance with a profile similar to isosceles triangle of 3-7 mm width and 3-6 mm height, which frontal wall (3) is arched and concaved by the radius of curvature of R=3-10 mm, **characterized in that** the hooked end comprising the workpiece frontal wall (3) is transverse and comprises the frontal cutting edge (5) and longitudinal cutting edges (4).

## Patentansprüche

1. Medizinisches Instrument zum Entfernen von Gelenkknorpel in Form eines Metallstabes, der auf der einen Seite mit einem Haltegriff (1) und auf der anderen Seite mit einem Arbeitswerkzeug (2) mit einer vorlaufenden Schneidekante (5) versehen ist, wobei die vorlaufende Schneidekante (5) an der vorlaufenden Eintrittskante und quer zu der Breite der Vorderwand (3) liegt, und das Arbeitswerkzeug (2) einen hakenförmigen Vorsprung mit einem Profil darstellt, welches einem gleichschenkligen Dreieck ähnelt und die Breite von 3-7 mm sowie Höhe von 3-6 mm aufweist, und dessen Vorderwand (3) mit einem Krümmungsradius R= 3-10 mm konkav gewölbt ist, **es zeichnet sich dadurch aus, daß** das hakenförmige Ende ein quer verlaufendes Vorderwand-Arbeitswerkzeug (3) beinhaltet sowie eine vorlaufende Schneidkante (5) und Längsschneidkanten (4).

## Revendications

1. Instrumenet médical d'élimination du cartilage articulaire sous la forme d'une tige métallique équipée d'un manche (1) d'un côté et d'un élément de travail (2) avec un bord tranchant (5) de l'autre côté, le bord tranchant (5) se trouve sur le bord tranchant et sur toute la largeur de la partie avant (3), et l'élément de travail (2) est une saillie en forme de crochet avec un profil similaire à un triangle isocèle de 3-7 mm de large et 3-6 mm de hauteur dont la paroi frontale (3) est bombée concave avec un rayon de courbure R = 3-10 mm, **caractérisé par son** extrémité en crochet qui comprend un élément de paroi frontale transversale (3), une arête frontale tranchante (5) et des arêtes longitudinales tranchantes (4).
